# EUROPÄISCHE PATENTSCHRIFT

(11) **EP 1 456 605 B1**
(45) Veröffentlichungstag und Bekanntmachung des Hinweises auf die Patenterteilung: **07.04.2010**
(21) Anmeldenummer: 02747306.5
(22) Anmeldetag: 07.05.2002
(51) Int. Cl.: G01C 11/02, G01B 11/24, A61B 5/107

(54) **VERFAHREN UND ANORDNUNG ZUR ERFASSUNG DER RAUMFORM EINES OBJEKTS**
METHOD AND SYSTEM FOR DETECTING THE THREE-DIMENSIONAL SHAPE OF AN OBJECT
PROCEDE ET SYSTEME POUR DETECTER LA FORME SPATIALE D'UN OBJET

(30) Priorität: 21.11.2001 DE 10156908
(43) Veröffentlichungstag der Anmeldung: 15.09.2004
(73) Patentinhaber: corpus.e AG, 70178 Stuttgart (DE)
(72) Erfinder: RUTSCHMANN, Dirk, 70179 Stuttgart (DE); JOSTEN, Marcus, 70190 Stuttgart (DE)
(74) Vertreter: Prinz & Partner
(86) Internationale Anmeldenummer: PCT/EP2002/005038
(87) Internationale Veröffentlichungsnummer: WO 2003/044464

(56) Entgegenhaltungen:
- EP-A- 0 958 782
- WO-A-94/20020
- WO-A-95/31934
- DE-A- 19 502 459
- DE-A- 19 634 254
- US-A- 4 535 782
- US-A- 6 108 497
- US-A- 6 155 120
- DATABASE WPI Section EI, Week 197948 Derwent Publications Ltd., London, GB; Class S02, AN 1979-L1916B XP002212184 & SU 652 440 A (CHERNII A N), 15. März 1979 (1979-03-15)

## Beschreibung

Die Erfindung betrifft ein Verfahren und eine Anordnung zur Erfassung der Raumform eines Objekts, z.B. eines menschlichen Beines.

Die berührungslose optische Erfassung der dreidimensionalen Raumform von Körpern und Körperteilen ist von grosser Bedeutung bei der automatisierten Maßanfertigung von Produkten, welche an die individuelle Körperform angepasst sein sollen, wie z.B. orthopädische Produkte, Maßschuhe, paßformgenaue Bekleidung usw. Die Kenntnis der Raumform ist auch von besonderer Bedeutung für die automatisierte Auswahl passender Produkte aus einem Katalog vorgefertigter Produkte. Als Beispiel sei die Auswahl einer passenden Fußeinlage aus einem großen Katalog vorgefertigter Einlagen mittels des individuellen 3D-Datensatzes der Fußform des Käufers genannt.

Zur Erfassung der Raumform sind eine große Anzahl von aufwendigen Abtastverfahren bekannt. In der EP 0 760 622 Bl wird z.B. von dem Erfinder Robert Massen ein besonders kostengünstiges Verfahren beschrieben, bei welchem der zu digitalisierende Körper mit einem elastischen, photogrammetrisch markierten Überzug versehen wird und dann aus einer Reihe sich überlappender Aufnahmepositionen photographiert wird. Hierbei braucht der Körperteil nicht in besonders genauer Weise zur Kamera ausgerichtet werden und es werden keine kalibrierten Aufnahmepositionen der Kamera oder Kameras benötigt, so daß die erforderlichen 2D-Aufnahmen sogar freihändig mit einer Digitalkamera durchgeführt werden können.

In der Patentanmeldung DE 100 25 922.7 vom gleichen Erfinder sind eine Reihe geeigneter Markierungsverfahren beschrieben, welche die automatische Findung homologer Bildpunkte in den einzelnen, sich überlappenden Aufnahmen ermöglichen und damit eine vollautomatische photogrammetrische Digitalisierung gestatten.

In der Patentanmeldung DE 100 49 926.0 vom gleichen Erfinder sind Kameras beschrieben, welche durch die Projektion von Zielmarken eine geordnete Sequenz von orientierten Aufnahmepositionen ermöglichen und damit die automatische Gewinnung homologer Bildpunkte weiter vereinfachen.

Aus DE-19634254 ist die photogrammetische Erfassung der Raumform eines Objekts bekannt, wobei ein Lichtmuster auf das Objekt projiziert wird und wobei Marken um die auf einer Fläche befindliche Auflagefläche des Objekts herum angeordnet sind.

Alle diese auf der Photogrammetrie basierenden Verfahren arbeiten mit am Körper befestigten Marken, was insbesondere dann nachteilig ist, wenn schlanke, durch einen elastischen Überzug markierte Körperteile aufgenommen werden sollen. Dann nämlich befinden sich alle Markierungen im wesentlichen in einem schmalen Ausschnitt in der Bildmitte der Aufnahmekamera. Dieses trifft für alle, sich überlappenden, aus einer Rundumsicht aufgenommenen Raumpositionen zu. Damit sind die homologen Bildpunkte ebenfalls in einem schmalen Ausschnitt jeder einzelnen Aufnahme konzentriert. Dies führt bei der photogrammetrischen Orientierung der einzelnen Aufnahmen zu großen Fehlern und Unsicherheiten, da die homologen Bildstrahlen sich in einem flachen Winkel schneiden und damit der Schnittpunkt nur ungenau im Raum definiert ist. Damit ist dann aber eine genaue Bestimmung der Raumform eines Objekts nicht in der gewünschten Weise möglich.

Darüber hinaus muß bei diesen bekannten Verfahren zur Gewinnung absoluter Raumkoordinaten in mindestens zwei Aufnahmen eine bekannte gerade Strecke vorhanden sein. Da die Markierungen auf dem elastischen Überzug keine festgelegte gerade Strecke im Raum festlegen, muß zusätzlich zu dem zu digitalisierenden Körper im Bildfeld ein bekannter starrer Maßstab eingebracht werden. Zur automatischen photogrammetrischen Auswertung muß dieser Maßstab automatisch in den einzelnen Aufnahmen erkannt und getrennt von den photogrammetrischen Markierungen des Körpers ausgewertet werden.

Die Aufgabe der Erfindung besteht darin, ein Verfahren und eine Anordnung zur photogrammetrischen Erfassung der Raumform eines Objekts zu schaffen, bei denen insbesondere bei schlanken Körpern eine genauere Erfassung der Raumform ermöglicht wird. Zudem wird gemäß der Erfindung der bei bisherigen Verfahren vorgesehene Maßstab überflüssig gemacht. Diese Aufgabe wird durch ein Verfahren gemäß Anspruchs und eine Vorrichtung gemäß Anspruch 9.

Da sich die die Anordnung auf der Fläche definierenden photogrammetrischen Markierungen so ausbilden lassen, daß sie im Bildfeld der einzelnen Aufnahmepositionen eines zu digitalisierenden schlanken Körpers weit weg von der der sich in der Bildmitte konzentrierenden erfaßten Markierungen des Körpers befinden, stellen sie sich in einem stumpfen Winkel schneidende homologe Strahlenbündel dar und ermöglichen so eine genaue photogrammetrische Orientierung der einzelnen Aufnahmepositionen. Dadurch lassen sich auch schlanke Körper mit hinreichender Genauigkeit digitalisieren.

Darüber hinaus kann die vorherbestimmte Anordnung von Marken auf der Fläche aber auch den bisher üblichen Maßstab ersetzen, da der definierte Abstand zwischen den photogrammetrischen Markierungen der auf der Fläche angebrachten Markenanordnung als Maßstab herangezogen werden kann.

Vorteilhafte Weiterbildungen der Erfindung sind in den abhängigen Patentansprüchen gekennzeichnet.

Weitere Merkmale und Vorteile der Erfindung ergeben sich aus der folgenden Beschreibung eines Ausführungsbeispiels anhand der Zeichnungen. In den Zeichnungen zeigen:
Fig. 1 eine bevorzugte Ausführungsform der Erfindung mit einem plattenförmigen Koordinatensystem, bei dem das zu erfassende Objekt der Unterkörper eines Menschen ist;
Fig. 2 den Bildbereich einer gemäß dem erfindungsgemäßen Verfahren erstellten Aufnahme des in der Fig. 2 dargestellten zu erfassenden Objekts.

Das erfindungsgemäße Verfahren bzw. die erfindungsgemäße Anordnung werden beispielshalber anhand der Erfassung der Raumform des Unterkörpers eines Menschen zwecks Anfertigung passgenauer Kompressionsstrümpfe beschrieben werden. Die Beschreibung dieses Beispiels ist jedoch nicht in einschränkender Weise zu verstehen.

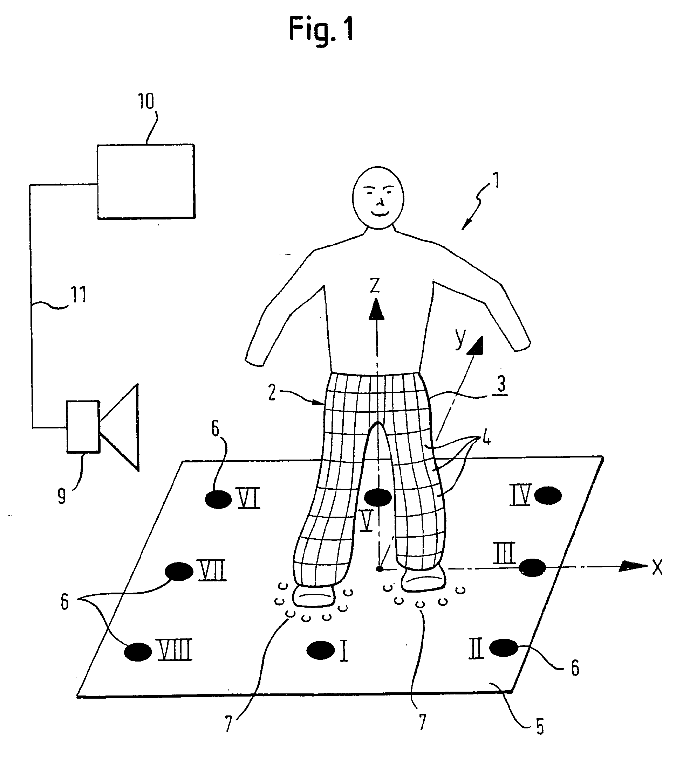

In der Figur 1 ist der Körper 1 eines Menschen zu erkennen. Es soll die dreidimensionale Form eines Teils des Körpers 1, nämlich des Unterkörpers 2 erfaßt werden. Hierzu ist der Unterkörper mit einem elastischen Überzug in Form einer Strumpfhose 3 versehen. Auf der Stumpfhose 3 sind photogrammetrische Marken in Form eines Gitternetzes angebracht, wobei die Schnittpunkte der Gitternetzlinien photogrammetrisch auswertbare Punktmarken 4 definieren.

Der Unterkörper 2 steht auf einer Platte 5, die ein zweidimensionales Koordinatensystem (x-y-Achsen) bildet. Auf der Platte sind photogrammetrisch auswertbare Punktmarken 6 angebracht, die das Koordinatensystem definieren. Die Punktmarken 6 sind dabei an vorherbestimmten Punkten auf der Platte 5 so angebracht, daß sie ein x-y-Koordinatensystem definieren. Die Punktmarken 6 sind um die Auflagefläche des Unterkörpers auf der Fläche 5 herum angeordnet. Diese Auflagefläche wird in der Fig. 1 durch die Schuhsohlen der die Strumpfhose tragenden Person definiert. Der Unterkörper 2 ist so auf der Platte angeordnet, daß er sich in einer bestimmten Ausrichtung zum x-y-Koordinatensystem befindet. Dabei sind auf der Platte weitere (nicht photogrammetrisch auswertbare) Markierungen 7 angebracht, die für die Füße der Person vorgesehen sind, so daß eine bestimmte Ausrichtung des Unterkörpers 2 zur Fläche gewährleistet ist. Der bekannte Abstand zwischen zwei beliebigen punktförmigen Plattenmarken 6 bildet eine gerade Strecke im Raum und stellt damit einen Maßstab zur Gewinnung absoluter Raumkoordinaten dar. Die durch die Plattenmarkierungen gebildete Ebene stellt z.B. die xy-Ebene eines weltkoordinatensystems dar, auf welchem sich der Körper des Patienten in etwa parallel zur Z-Achse ausgerichtet befindet. Der Nullpunkt dieses Koordinatensystems wird zweckmäßigerweise ebenfalls anhand der Plattenmarkierungen festgelegt, z.B. in der Mitte zwischen zwei sich gegenüberliegenden photogrammetrischen Marken.

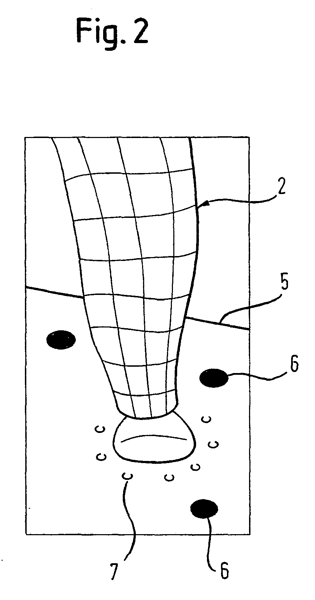

Zunächst werden nun mehrere Aufnahmen des zu erfassenden Unterkörpers 2 aus verschiedenen Ansichten erstellt. Die sich überlappenden Bildaufnahmepositionen werden dabei so gewählt, daß zumindestens in einigen der einzelnen Aufnahmen neben dem markierten Ausschnitt des Unterkörpers 2 ebenfalls ein Ausschnitt der markierten Platte 5 zu erkennen ist. Dieses ist in der Fig. 2 dargestellt, die exemplarisch einen Bildausschnitt zeigt. Dabei sind für sich entsprechende Elemente dieselben Bezugszeichen verwendet worden wie in der Fig. 1.

Da die Plattenmarkierungen 6 sich im Bildfeld der einzelnen Aufnahmepositionen weit weg von den sich in der Bildmitte konzentrierenden Markierungen des zu digitalisierenden Beines befinden, stellen sie sich in einem stumpfen Winkel schneidende Strahlenbündel dar und ermöglichen so eine genaue photogrammetrische Orientierung der einzelnen Aufnahmepositionen. Damit sind erfindungsgemäß alle oben beschriebenen Nachteile der bekannten sehr kostengünstigen Verfahren beseitigt.

Gleichzeitig definiert die Platte 5 einen einfachen Messraum, in dem, z.B. im orthopädischen Fachgeschäft, in der Arztpraxis usw. die erforderlichen Rundum-Aufnahmen wohlgeordnet durchgeführt werden können. Durch weitere nicht-photogrammetrische Marken wie z.B. den einzelnen Aufnahmepositionen zugeordnete, durchnummerierte Zielmarken kann der die Aufnahmen durchführende Operator so geführt werden, dass die einzelnen überlappenden Bilder in einer vorbestimmten Reihenfolge aufgenommen werden. In der Fig. 1 ist das durch römische Ziffern dargestellt.

Solche Zielmarken sind auch nützlich, um die mit einem Lichtmarkenprojektor versehene Aufnahmekamera 9 vordefiniert auszurichten. Erfindungsgemäß werden diese nichtphotogrammetrischen Marken optisch durch Farb-, Textur- oder Formgebung oder durch das Reflexionsverhalten so gestaltet, daß sie mit Verfahren der Bildverarbeitung automatisch von den photogrammetrischen Marken 4 und 6 unterschieden werden können.

Die in der oben beschriebenen Weise erstellten Aufnahmen werden dann mit einem bekannten System 10 zur Auswertung der Aufnahmen und zur Ermittlung der Raumform ausgewertet und es wird anhand der homologen auf dem Objekt und zu dem Koordinatensystem gehörenden Marken 4 bzw. 6 die Raumform des Objekts ermittelt. Hierbei kann z.B. ein Computer eingesetzt werden, der mit einer entsprechenden Software programmiert wurde.

Handelt es sich bei dem Aufnahmesystem 9 um eine Digitalkamera, so können die digitalisierten Aufnahmen in besonderes einfacher Form über eine Leitungsverbindung 11 oder auch drahtlos an das Auswertungssystem 10 übermittelt werden.

Gemäß der Erfindung ist die markierte Platte 5 mit weiteren, für den jeweiligen Zweck nützlichen Meßfunktionen ausgestattet. So kann z.B. die Platte 5 eine Waage enthalten, um gleichzeitig neben der Raumform das Gewicht des Patienten zu ermitteln. Die Platte kann auch einen Fußdrucksensor enthalten, um die Druckverteilung der Fußsohle bzw. die ungleiche Druckverteilung auf beiden Füßen infolge der Körperasymmetrie zu messen.

Die Platte 5 kann darüber hinaus auch Sensoren enthalten, um die Temperaturverteilung, die Feuchtigkeitsverteilung usw. der Fußsohlen zu ermitteln.

Gemäß einer weiteren Ausführungsform kann die Platte 5 auch Aktuatoren enthalten, um bestimmte Positionen des zu digitalisierenden Körperteils zu erzwingen. So kann z.B. durch einen motorisch den Fuß aufstellenden Hebel dieser vor dem Digitalisieren in eine anatomisch gewünschte Stellung wie z.B. auf dem Fußballen aufstehender Fuß, gebracht werden.

Erfindungsgemäß kann die starre Fläche 5 auch gekrümmt sein. So ist es z.B. nützlich, zur Digitalisierung eines liegenden Oberkörpers eine zylindrische, photogrammetrisch markierte Auflagefläche zu verwenden, welche ein zylinderförmiges Weltkoordinatensystem definiert.

Ein weiterer Erfindungsgedanke ist es, ein Material für die Fläche der Platte 5 zu verwenden, welches sich unter der Last des Körperteils verformt und dass aus der photogrammetrisch zusätzlich zur Raumform des Körperteils gemessenen räumlichen Verformung der Fläche auf physikalische Eigenschaften des zu digitalisierenden Körpers geschlossen werden kann. So kann z.B. aus der Verformung der starren Platte aus auf das Gewicht des Körpers geschlossen werden, ohne dass hierzu eine Waage benötigt wird. Die Verformung ergibt sich nämlich durch die Verschiebung der Punktmarken 6, die auf der Platte angebracht sind. Diese Verschiebung läßt sich im Zuge der photogrammetrischen Auswertung der Aufnahmen ermitteln.

Es ist für den Fachmann ersichtlich, daß das oben beschriebene Ausführungsbeispiel natürlich auch dahingehend abgewandelt werden kann, daß das zweidimensionale Koordinatensystem auf der Fläche (5) durch eine beliebige vorherbestimmte Anordnung von photogrammetrisch auswertbaren Marken ersetzt wird. Wichtig ist nur, daß diese Anordnung eine Ausdehnung aufweist, bei der die weiteren Marken 6 der Anordnung um die auf der Fläche befindliche Auflagefläche des Objekts herum angeordnet sind.

## Patentansprüche

1. Verfahren zur Erfassung der Raumform und weiterer physikalischen Eigenschaften eines Objekts, bei dem
ein mit photogrammetrisch auswertbaren Marken (4) versehenes Objekt (2) an einer vorherbestimmten Stelle auf eine Fläche aufgebracht wird, die mit weiteren photogrammetrisch auswertbaren Marken (6) versehen ist, die in vorherbestimmter Weise zueinander angeordnet sind, wobei die Fläche eine solche Ausdehnung aufweist, dass die weiteren Marken (6) um die auf der Fläche befindliche Auflagefläche des Objekts (2) herum angeordnet sind,
mehrere Aufnahmen des Objekts (2) aus verschiedenen Ansichten in der Weise gemacht werden, dass darin neben dem Objekt (2) auch die Fläche (5) zumindest teilweise mit abgebildet wird, und
aus den Aufnahmen unter Zuhilfenahme der Marken (4) des Objekts und der weiteren Marken (6) der Fläche (2) mit einem photogrammetrischen Verfahren die Raumform des Objekts (2) ermittelt wird; **dadurch gekennzeichnet, dass** die Fläche von einer Platte (5) gebildet wird, die Sensoren enthält, die weitere physikalische Eigenschaften des Objekts (2) erfassen.

2. Verfahren nach Anspruch 1, **dadurch gekennzeichnet, dass** die Sensoren mindestens eine der folgenden Größen erfassen;
- Gewicht einer auf der Platte stehenden Person;
- Druckverteilung der Fußsohle bzw. die ungleiche Druckverteilung auf beiden Füßen infolge der Körperasymmetrie einer auf der Platte stehenden Person;
- Temperaturverteilung der Fußsohlen einer auf der Platte stehenden Person;
- Feuchtigkeitsverteilung der Fußsohlen einer auf der Platte stehenden Person.

3. Verfahren nach Anspruch 1 oder 2, bei dem die weiteren photogrammetrisch auswertbaren Marken (6) der Fläche (2) so zueinander angeordnet sind, dass sie ein zweidimensionales Koordinatensystem bilden.

4. Verfahren nach Anspruch 3, bei dem das Koordinatensystem durch schwarze Punktmarken (6) definiert wird, die in bestimmten Abständen zueinander auf der Fläche (5) angebracht sind.

5. Verfahren nach einem der vorhergehenden Ansprüche, bei dem auf der Fläche (5) weitere Markierungen (7) angebracht werden, welche die Ausrichtung des Objekts (2) auf der Fläche erleichtern.

6. Verfahren nach einem der vorhergehenden Ansprüche, bei dem die Fläche (5) so ausgebildet ist, dass sie sich bei Aufbringen des Objekts (2) auf die Fläche (5) verformt.

7. Verfahren nach Anspruch 6, bei dem mit Hilfe des photogrammetrischen Verfahrens die Verformung der Fläche (5) ermittelt wird und daraus physikalische Eigenschaften des Objekts (2) berechnet werden.

8. Verfahren nach einem der vorhergehenden Ansprüche, bei dem das Objekt (2) ein menschlicher Körper oder ein menschliches Körperteil ist.

9. Anordnung zur Erfassung der Raumform und weiterer physikalischen Eigenschaften eines Objekts mit photogrammetrisch auswertbaren Marken (4), die auf dem Objekt (2) angebracht sind, mit einer Fläche, die mit weiteren photogrammetrisch auswertbaren Marken (6) versehen ist, die in vorherbestimmter Weise zueinander angeordnet sind, wobei die Fläche (5) eine solche Ausdehnung aufweist, dass die weiteren Marken (6) um die auf der Fläche befindliche Auflagefläche des Objekts (2) herum angeordnet sind, einem Aufnahmesystem (9) und einem System (10) zur Auswertung der Aufnahmen und zur Ermittlung der Raumform, **dadurch gekennzeichnet, dass** die Fläche auf einer Platte (5) gebildet ist, die Sensoren enthält, womit weitere physikalische Eigenschaften des Objekts (2) erfassbar sind.

10. Anordnung nach Anspruch 9, **dadurch gekennzeichnet, dass** die Sensoren zur Erfassung mindestens einer der folgenden Größen ausgebildet sind:
- Gewicht einer auf der Platte stehenden Person;
- Druckverteilung der Fußsohle bzw. die ungleiche Druckverteilung auf beiden Füßen infolge der Körperasymmetrie einer auf der Platte stehenden Person;
- Temperaturverteilung der Fußsohlen einer auf der Platte stehenden Person;
- Feuchtigkeitsverteilung der Fußsohlen einer auf der Platte stehenden Person.

11. Anordnung nach Anspruch 9 oder 10, bei der das System (10) zur Auswertung der Aufnahmen und zur Ermittlung der Raumform so ausgebildet ist, dass unter Zuhilfenahme der Marken (4) des Objekts und der Marken (6) der Fläche (5) die Raumform des Objekts (2) ermittelbar ist.

12. Anordnung nach Anspruch 9, 10 oder 11, bei der die weiteren photogrammetrisch auswertbaren Marken (6) der Fläche (2) so zueinander angeordnet sind, dass sie ein zweidimensionales Koordinatensystem bilden.

13. Anordnung nach Anspruch 12, bei der das Koordinatensystem durch schwarze Punktmarken (6) definiert wird, die in bestimmten Abständen zueinander auf der Fläche (5) angebracht sind.

14. Anordnung nach einem der Ansprüche 9 bis 13, bei der auf der Fläche (5) weitere Markierungen (7) angebracht sind, zum Erleichtern der Ausrichtung des Objekts (2) auf der Fläche (5).

15. Anordnung nach einem der Ansprüche 9 bis 14, bei der die Fläche (5) so ausgebildet ist, dass sie sich bei Aufbringen des Objekts (2) auf die Fläche (5) verformt.

16. Anordnung nach Anspruch 15, bei der mit Hilfe des Systems (10) zur Auswertung der Aufnahmen und zur Ermittlung der Raumform die Verformung der Fläche (5) ermittelbar ist und daraus physikalische Eigenschaften des Objekts (2) berechenbar sind.

17. Anordnung nach einem der Ansprüche 9 bis 16, bei der das Objekt (2) ein menschlicher Körper oder ein menschliches Körperteil ist.

18. Verfahren nach einem der Ansprüche 1-8 oder Anordnung nach einem der Ansprüche 9-17, bei dem bzw. der das Objekt (2) mit einem Überzug (3) versehen ist, auf dem die photogrammetrisch auswertbaren Marken (4) angebracht sind.

## Claims

1. A method of detecting the three-dimensional shape and further physical properties of an object, in which
an object (2) provided with photogrammetrically evaluable marks (4) is placed at a predetermined location on a surface that is provided with further photogrammetrically evaluable marks (6) arranged in a predetermined manner with respect to each other, the surface having an extent such that the further marks (6) are arranged around the contact surface, located on the surface, of the object (2),
several pictures of the object (2) are taken from different views in such a way that, in addition to the object (2), the surface (5) is also at least partially depicted therein, and
the three-dimensional shape of the object (2) is determined from the pictures with the aid of the marks (4) of the object and the further marks (6) of the surface (2) by using a photogrammetric method; **characterized in that** the surface is formed by a plate (5) containing sensors that detect further physical properties of the object (2).

2. The method according to claim 1, **characterized in that** the sensors detect at least one of the following quantities:
- weight of a person standing on the plate;
- pressure distribution of the sole of the foot or the uneven pressure distribution on both feet as a result of body asymmetry of a person standing on the plate;
- temperature distribution of the soles of the feet of a person standing on the plate;
- moisture distribution of the soles of the feet of a person standing on the plate.

3. The method according to claim 1 or 2, in which the further photogrammetrically evaluable marks (6) of the surface (2) are arranged with respect to each other in such a way that they form a two-dimensional coordinate system.

4. The method according to claim 3, in which the coordinate system is defined by black dot marks (6) that have been applied on the surface (5) at specific intervals from each other.

5. The method according to any of the preceding claims, in which further markings (7) are applied onto the surface (5), which facilitate the orientation of the object (2) on the surface.

6. The method according to any of the preceding claims, in which the surface (5) is configured in such a way that it deforms when the object (2) is placed onto the surface (5).

7. The method according to claim 6, in which the deformation of the surface (5) is determined with the aid of the photogrammetric method and physical properties of the object (2) are computed therefrom.

8. The method according to any of the preceding claims, in which the object (2) is a human body or a part of a human body.

9. An arrangement for detecting the three-dimensional shape and further physical properties of an object by photogrammetrically evaluable marks (4) that have been applied on the object (2), with a surface that is provided with further photogrammetrically evaluable marks (6) arranged in a predetermined manner with respect to each other, the surface (5) having an extent such that the further marks (6) are arranged around the contact surface, located on the surface, of the object (2), a shooting system (9) and a system (10) for evaluating the pictures and for determining the three-dimensional shape, **characterized in that** the surface is formed on a plate (5) containing sensors with which further physical properties of the object (2) are detectable.

10. The arrangement according to claim 9, **characterized in that** the sensors are configured for detecting at least one of the following quantities:
- weight of a person standing on the plate;
- pressure distribution of the sole of the foot or the uneven pressure distribution on both feet as a result of body asymmetry of a person standing on the plate;
- temperature distribution of the soles of the feet of a person standing on the plate;
- moisture distribution of the soles of the feet of a person standing on the plate.

11. The arrangement according to claim 9 or 10, in which the system (10) for evaluating the pictures and for determining the three-dimensional shape is configured in such a way that the three-dimensional shape of the object (2) can be determined with the aid of the marks (4) of the object and the marks (6) of the surface (5).

12. The arrangement according to claim 9, 10, or 11, in which the further photogrammetrically evaluable marks (6) of the surface (2) are arranged with respect to each other in such a way that they form a two-dimensional coordinate system.

13. The arrangement according to claim 12, in which the coordinate system is defined by black dot marks (6) that have been applied onto the surface (5) at specific intervals from each other.

14. The arrangement according to any of claims 9 to 13, in which further markings (7) have been applied on the surface (5), for facilitating the orientation of the object (2) on the surface (5).

15. The arrangement according to any of claims 9 to 14, in which the surface (5) is configured in such a way that it deforms when the object (2) is placed onto the surface (5).

16. The arrangement according to claim 15, in which the deformation of the surface (5) can be determined with the aid of the system (10) for evaluating the pictures and for determining the three-dimensional shape, and physical properties of the object (2) are computable therefrom.

17. The arrangement according to any of claims 9 to 16, in which the object (2) is a human body or a part of a human body.

18. A method according to any of claims 1 to 8 or an arrangement according to any of claims 9 to 17, in which the object (2) is provided with a covering (3) onto which the photogrammetrically evaluable marks (4) have been applied.

## Revendications

1. Procédé de détection de la forme spatiale et d'autres propriétés physiques d'un objet, dans lequel
un objet (2) pourvu de repères (4) analysables par photogrammétrie est placé à un endroit prédéterminé sur une surface pourvue d'autres repères (6) analysables par photogrammétrie qui sont agencés de manière prédéterminée les uns par rapport aux autres, la surface présentant une telle étendue que les autres repères (6) sont agencés autour de la surface de contact de l'objet (2), qui se trouve sur la surface,
plusieurs prises de vue de l'objet (2) sont faites dans différentes vues de telle manière qu'en plus de l'objet (2), la surface (5) est également représentée au moins partiellement, et
la forme spatiale de l'objet (2) est déterminée à partir des prises de vue à l'aide des repères (4) de l'objet et des autres repères (6) de la surface (2) en utilisant un procédé photogrammétrique, **caractérisé en ce que** la surface est formée par une plaque (5) comportant des capteurs qui détectent d'autres propriétés physiques de l'objet (2).

2. Procédé selon la revendication 1, **caractérisé en ce que** les capteurs détectent au moins l'une des grandeurs suivantes :
- le poids d'une personne debout sur la plaque ;
- la distribution de la pression de la plante des pieds ou la distribution irrégulière de la pression sur les deux pieds en raison de l'asymétrie du corps d'une personne debout sur la plaque ;
- la distribution de la température de la plante des pieds d'une personne debout sur la plaque ;
- la distribution de l'humidité de la plante des pieds d'une personne debout sur la plaque.

3. Procédé selon la revendication 1 ou 2, dans lequel les autres repères (6) de la surface (2) qui sont analysables par photogrammétrie sont agencés de telle sorte les uns par rapport aux autres qu'ils forment un système de coordonnées bidimensionnel.

4. Procédé selon la revendication 3, dans lequel le système de coordonnées est défini par des repères ponctuels (6) noirs qui sont agencés sur la surface (5) à intervalles déterminés les uns par rapport aux autres.

5. Procédé selon l'une des revendications précédentes, dans lequel d'autres marques (7) sont placées sur la surface (5), lesquelles facilitent l'orientation de l'objet (2) sur la surface.

6. Procédé selon l'une des revendications précédentes, dans lequel la surface (5) est réalisée de telle sorte qu'elle se déforme lorsque l'objet (2) est disposé sur la surface (5).

7. Procédé selon la revendication 6, dans lequel la déformation de la surface (5) est déterminée à l'aide du procédé photogrammétrique et des propriétés physiques de l'objet (2) sont calculées à partir de celle-ci.

8. Procédé selon l'une des revendications précédentes, dans lequel l'objet (2) est un corps humain ou une partie du corps humain.

9. Agencement pour la détection de la forme spatiale et d'autres propriétés physiques d'un objet à l'aide de repères (4) analysables par photogrammétrie qui sont agencés sur l'objet (2), avec une surface pourvue d'autres repères (6) analysables par photogrammétrie qui sont agencés de manière prédéterminée les uns par rapport aux autres, la surface (5) présentant une telle étendue que les autres repères (6) sont agencés autour de la surface de contact de l'objet (2), qui se trouve sur la surface, un système de prise de vue (9) et un système (10) pour l'évaluation des prises de vue et la détermination de la forme spatiale, **caractérisé en ce que** la surface est réalisée sur une plaque (5) comportant des capteurs à l'aide desquels d'autres propriétés physiques de l'objet (2) peuvent être détectées.

10. Agencement selon la revendication 9, **caractérisé en ce que** les capteurs sont réalisés pour la détection d'au moins l'une des grandeurs suivantes :
- le poids d'une personne debout sur la plaque ;
- la distribution de la pression de la plante des pieds ou la distribution irrégulière de la pression sur les deux pieds en raison de l'asymétrie du corps d'une personne debout sur la plaque ;
- la distribution de la température de la plante des pieds d'une personne debout sur la plaque ;
- la distribution de l'humidité de la plante des pieds d'une personne debout sur la plaque.

11. Agencement selon la revendication 9 ou 10, dans lequel le système (10) pour l'évaluation des prises de vue et la détermination de la forme spatiale est réalisé de telle sorte que la forme spatiale de l'objet (2) peut être déterminée à l'aide des repères (4) de l'objet et des repères (6) de la surface (5).

12. Agencement selon la revendication 9, 10 ou 11, dans lequel les autres repères (6) de la surface (2) qui sont analysables par photogrammétrie sont agencés de telle sorte les uns par rapport aux autres qu'ils forment un système de coordonnées bidimensionnel.

13. Agencement selon la revendication 12, dans lequel le système de coordonnées est défini par des repères ponctuels (6) noirs qui sont agencés sur la surface (5) à intervalles déterminés les uns par rapport aux autres.

14. Agencement selon l'une des revendications 9 à 13, dans lequel d'autres marques (7) sont placées sur la surface (5) afin de faciliter l'orientation de l'objet (2) sur la surface (5).

15. Agencement selon l'une des revendications 9 à 14, dans lequel la surface (5) est réalisée de telle sorte qu'elle se déforme lorsque l'objet (2) est disposé sur la surface (5).

16. Agencement selon la revendication 15, dans lequel la déformation de la surface (5) peut être déterminée à l'aide du système (10) pour l'évaluation des prises de vue et la détermination de la forme spatiale, et des propriétés physiques de l'objet (2) peuvent être calculées à partir de celle-ci.

17. Agencement selon l'une des revendications 9 à 16, dans lequel l'objet (2) est un corps humain ou une partie du corps humain.

18. Procédé selon l'une des revendications 1 à 8 ou agencement selon l'une des revendications 9 à 17, dans lequel l'objet (2) est pourvu d'un revêtement (3) sur lequel sont placés les repères (4) analysables par photogrammétrie.
